# EUROPÄISCHE PATENTSCHRIFT

(11) **EP 1 387 840 B1**
(45) Veröffentlichungstag und Bekanntmachung des Hinweises auf die Patenterteilung: **15.12.2004**
(21) Anmeldenummer: 02771564.8
(22) Anmeldetag: 24.04.2002
(51) Int. Cl.: C07D 401/14, C07D 471/04, A61K 31/4196, A61P 35/00

(54) **3,5-DIAMINO-1,2,4-TRIAZOLE ALS KINASE INHIBITOREN**
3,5-DIAMINO-1,2,4-TRIAZOLES AS KINASE INHIBITORS
3,5-DIAMINO-1,2,4-TRIAZOLES EN TANT QU'INHIBITEURS DE KINASE

(30) Priorität: 08.05.2001 DE 10123586
(43) Veröffentlichungstag der Anmeldung: 11.02.2004
(73) Patentinhaber: Schering Aktiengesellschaft, 13342 Berlin (DE)
(72) Erfinder: KRÜGER, Martin, 13465 Berlin (DE); PETROV, Orlin, 14199 Berlin (DE); THIERAUCH, Karl-Heinz, 14169 Berlin (DE); SIEMEISTER, Gerhard, 13503 Berlin (DE)
(86) Internationale Anmeldenummer: PCT/EP2002/004559
(87) Internationale Veröffentlichungsnummer: WO 2002/094814

(56) Entgegenhaltungen:
- WO-A-00/10563
- WO-A-00/25780

## Beschreibung

Die vorliegende Erfindung betrifft 3,5-Diamino-1,2,4-triazole als Kinase Inhibitoren, deren Herstellung sowie deren Verwendung als Medikament zur Behandlung verschiedener Erkrankungen.

Angiogenesehemmung ist ein möglicher Behandlungsmodus für Tumorerkrankungen. Dabei wird die Versorgung des wachsenden Tumors durch eine Hemmung des gleichmäßigen Mitwachsens des Blutgefäßsystems verschlechtert. Dies führt mindestens zu verlangsamtem Wachstum, zur Stabilisierung des bestehenden Zustandes oder häufig zur Regression. Als wichtige Signalgeber für das Gefäßwachstum sind die Wachstumsfaktoren VEGF (vascular endothelial growth factor) und für die Gefäßstabilisierung PDGF und Tie2 erkannt worden. Die jeweiligen Gen-Knock-outs der Wachstumsfaktoren und ihrer Rezeptoren führten zu einem letalen vaskulären Phänotyp im Embryonalstadium. Tumore sind charakterisiert durch beschleunigtes Zellwachstum im Vergleich zum Normalgewebe. Die Wachstumshemmung und die folgende Apoptose von Tumorzellen ist ein Prinzip vieler Zytostatika und zytotoxischer Arzneimittel mit Tumorindikationen. Durch das vertiefte Wissen, das in der Biologie über die Mechanismen der Proliferation erarbeitet worden ist, ist es gelungen, wesentliche Regulatoren des Zellzyklus zu identifizieren, die bei der Zellteilung aktiviert werden. Ganz wichtig sind die Rollen der CDK's (cell cycle dependent kinases), die als zentrale Schaltstellen in den verschiedenen Phasen der Zellteilung funktionieren. Gelingt es, die Hemmung der Signalübertragung, die für eine geordnete Blutgefäßbildung notwendig ist, mit der Hemmung des Zellzyklus zu verbinden, der sowohl für Tumor als auch für Blutgefäßwandzellen Voraussetzung ist, dann tritt eine Tumorwachstumshemmung auf, die wesentlich weiter geht, als würde man nur einen der beiden Prozesse hemmen.

Es ist bereits bekannt, daß 1,2,4-Triazole als Inhibitoren der Inosin-Monophosphat-Dehydrogenase (IMPDH) zum Einsatz kommen und damit als Immunsuppressoren, anti-Krebsmittel, antivaskulare hyperproliferative Verbindungen, Entzündungshemmer, antimykotische, antipsoriatische und antivirale Verbindungen Verwendung finden (WO 00/25780).

Deren Wirkung als Kinase-Hemmer ist jedoch nicht bekannt.

Aufgrund der interessanten Eigenschaften der Verbindungsklasse besteht nach wie vor ein großer Bedarf an selektiveren und insbesondere wirksameren Verbindungen zur Behandlung von verschiedenen Erkrankungen.

Es wurde nun gefunden, daß Verbindungen der allgemeinen Formel I 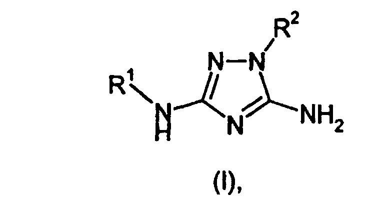 in der
- R¹: für mono- oder bicyclisches Heteroaryl, welches gegebenfalls ein- oder mehrfach, gleich oder verschieden substituiert sein kann und
- R²: für mono- oder bicyclisches Aryl oder Heteroaryl, welches gegebenfalls ein- oder mehrfach, gleich oder verschieden substituiert sein kann, stehen, sowie deren Isomeren und Salze,
einerseits eine hohe Potenz bei der Signalübertragung für angiogeneserelevante Prozesse durch Blockade der Rezeptortyrosinkinasen aufweisen und andererseits die Zellproliferation durch eine Hemmung der Serin/Threonin-Zellzykluskinasen beeinflussen.

Folglich lassen sich die erfindungsgemäßen Verbindungen für die Behandlung verschiedenster Geschwulsterkrankungen unter Einschluß hämangiogenetischer proliferativer Erkrankungen verwenden. Hierzu zählt zum Beispiel Krebs, wie solide Tumoren und Leukämie, Autoimmunerkrankungen wie Psoriasis, Alopezie und Multiple Sklerose, Chemotherapeutika-induzierte Alopezie und Mukositis, kardiovaskulare Erkrankungen, wie Stenosen, Arteriosklerosen und Restenosen, infektiöse Erkrankungen, wie z. B. durch unizellulare Parasiten, wie Trypanosoma, Toxoplasma oder Plasmodium, oder durch Pilze hervorgerufen, nephrologische Erkrankungen, wie z. B. Glomerulonephritis, chronische neurodegenerative Erkrankungen, wie Huntington's Erkrankung, amyotropisch laterale Sklerose, Parkinsonsche Erkrankung, AIDS Dementia und Alzheimer'sche Erkrankung, akute neurodegenerative Erkrankungen, wie Ischämien des Gehirns und Neurotraumata, virale Infektionen, wie z. B. Cytomegalus-Infektionen, Herpes, Hepatitis B und C, und HIV Erkrankungen.

Als besonders wertvoll haben sich solche Verbindungen der allgemeinen Formel I erwiesen, in der
- R¹: für die Gruppe 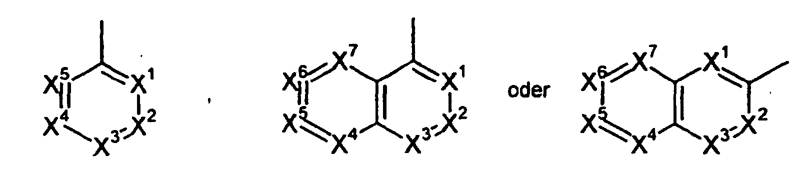 steht, worin
- X¹ bis X⁷: unabhängig voneinander für ein Stickstoff-Atom oder für die Gruppe CR³ stehen, wobei mindestens ein X im Ring für ein Stickstoff-Atom steht und
- R³: für Wasserstoff, Hydroxy, Halogen, C₁-C₁₀-Alkyl, C₁-C₁₀-Alkoxy, Halogen-C₁-C₁₀-alkyl, Halogen-C₁-C₁₀-alkoxy, Aryl, Aryloxy, Heteroaryl, Heteroaryloxy oder für die Gruppe -NR⁴R⁵ steht,
- R²: für die Gruppe 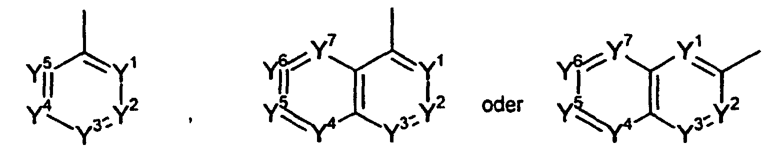 steht, worin
- Y¹ bis Y⁷: unabhängig voneinander für ein Stickstoff-Atom oder für die Gruppe CR⁶ stehen und
- R⁶: für Wasserstoff, Hydroxy, Halogen, Nitro, Cyano, C₁-C₁₀-Alkyl, C₁-C₁₀- Alkoxy, Halogen-C₁-C₁₀-alkyl, Halogen-C₁-C₁₀-alkoxy, Aryl, Aryloxy, Heteroaryl, Hetaroaryloxy, C₁-C₁₀-Alkylcarbonyl, C₁-C₁₀-Alkoxycarbonyl oder für die Gruppe -NR⁴R⁵ steht, bedeuten, sowie deren Isomeren und Salze.

Unter Alkyl ist jeweils ein geradkettiger oder verzweigter Alkylrest, wie beispielsweise Methyl, Ethyl, Propyl, Isopropyl, Butyl, Isobutyl, sek. Butyl, tert. Butyl, Pentyl, Hexyl, Heptyl, Octyl, Nonyl oder Decyl zu verstehen, wobei C₁₋₆-Alkylreste bevorzugt werden.

Unter Alkoxy ist jeweils ein geradkettiger oder verzweigter Alkoxyrest, wie beispielsweise Methyloxy, Ethyloxy, Propyloxy, Isopropyloxy, Butyloxy, Isobutyloxy, sek. Butyloxy, tert. Butyloxy, Pentyloxy, Hexyl, Heptyloxy, Octyloxy, Nonyloxy oder Decyloxy zu verstehen, wobei C₁₋₆-Alkoxyreste bevorzugt werden.

Unter Halogen ist jeweils Fluor, Chlor, Brom oder Jod zu verstehen.

Der Arylrest hat jeweils 6 - 12 Kohlenstoffatome wie beispielsweise Naphthyl, Biphenyl und insbesondere Phenyl.

Der Heteroarylrest kann jeweils benzokondensiert sein. Beispielsweise seien als 5-Ringheteroaromaten genannt: Thiophen, Furan, Oxazol, Thiazol, Imidazol und Benzoderivate davon und als 6-Ring-Heteroaromaten Pyridin, Pyrimidin, Triazin, Chinolin, Isochinolin und Benzoderivate davon.
Ist eine saure Funktion enthalten, sind als Salze die physiologisch verträglichen Salze organischer und anorganischer Basen geeignet, wie beispielsweise die gut löslichen Alkali- und Erdalkalisalze sowie N-Methyl-glukamin, Dimethyl-glukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten, sind die physiologisch verträglichen Salze organischer und anorganischer Säuren geeignet wie Salzsäure, Schwefelsäure, Phosphorsäure, Zitronensäure, Weinsäure u.a.

Als besonders wirksam haben sich solche Verbindungen der allgemeinen Formel I erwiesen, in der
- R¹: für die Gruppe 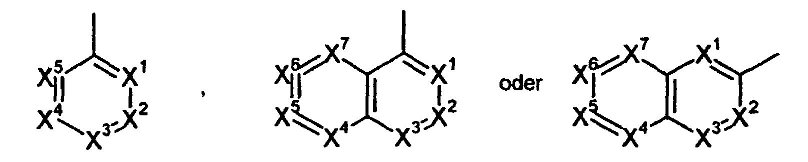 steht, worin
- X¹ bis X⁷: unabhängig voneinander für ein Stickstoff-Atom oder für die Gruppe CR³ stehen, wobei mindestens ein X im Ring für ein Stickstoff-Atom steht und
- R³: für Wasserstoff, Halogen, C₁-C₆-Alkyl oder C₁-C₆-Alkoxy steht,
- R²: für die Gruppe 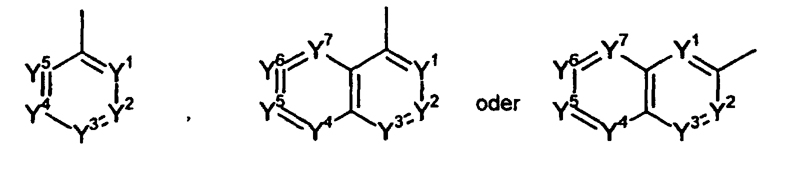 steht, worin
- Y¹ bis Y⁷: unabhängig voneinander für ein Stickstoff-Atom oder für die Gruppe CR⁶ stehen und
- R⁶: für Wasserstoff, C₁-C₆-Alkyl oder Halogen-C₁-C₆-alkyl steht, bedeuten, sowie deren Isomeren und Salze.

Als ganz besonders wirksam haben sich solche Verbindungen der allgemeinen Formel I erwiesen, in der
- R¹: für die Gruppe 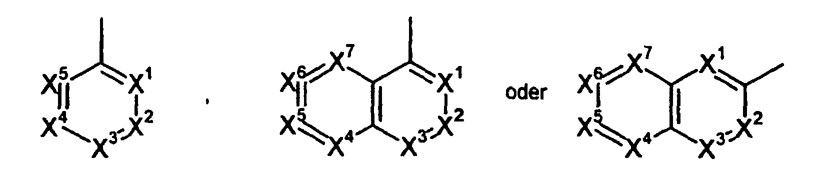 steht, worin
- X¹ bis X⁴: unabhängig voneinander für ein Stickstoff-Atom oder für die Gruppe CR³ stehen, wobei mindestens ein X für ein Stickstoff-Atom steht,
- X⁵ bis X⁷: für die Gruppe CR³ stehen,
- R³: für Wasserstoff, Methoxy, Brom, Chlor oder Methyl steht,
- R²: für die Gruppe 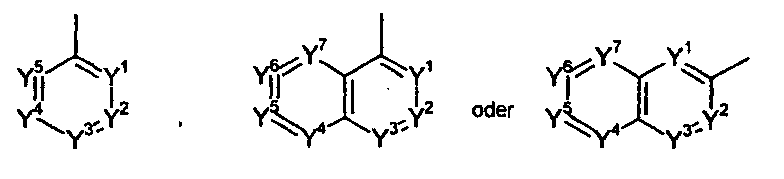 steht, worin
- Y¹ bis Y⁷: unabhängig voneinander für ein Stickstoff-Atom oder für die Gruppe CR⁶ stehen und
- R⁶: für Wasserstoff, Methyl oder Trifluormethyl steht, bedeuten, sowie deren Isomeren und Salze.

### Herstellung der erfindungsgemäßen Verbindungen

Die nachfolgenden Beispiele erläutern die Herstellung der erfindungsgemäßen Verbindungen, ohne den Umfang der beanspruchten Verbindungen auf diese Beispiele zu beschränken.

Die erfindungsgemäßen Verbindungen lassen sich nach literaturbekannten Methoden gemäß folgender Reaktion herstellen (z.B.: J. Heterocyclic Chem., **19,** 1205 (1982) und J. Heterocyclic Chem., **24,** 275 (1987)):

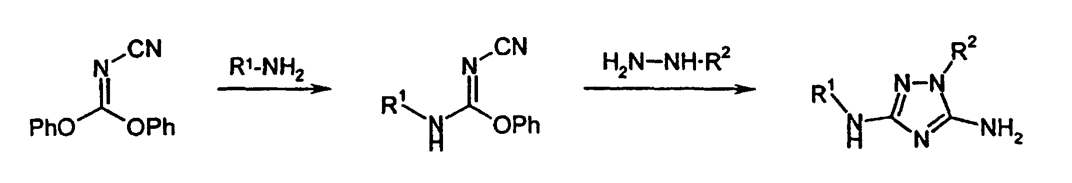

Ein Hetarylamin kann in einem geeigneten Lösungsmittel wie z.B. Ethanol, Isopropanol, Dioxan oder Acetonitril mit dem käuflichen Diphenylcyanocarbonimidat bei einer Temperatur zwischen 20 °C und der Siedetemperatur des Lösungsmittels umgesetzt werden. Der erhaltene N-Cyano-O-phenyl-isoharnstoff wird in einem geeigneten Lösungsmittel wie z.B. Methanol mit einem entsprechend substituierten Hydrazin bei einer Temperatur zwischen 20 °C und der Siedetemperatur des Lösungsmittels zu den erfindungsgemäßen 3,5-Diamino-1,2,4-triazol-Derivaten umgesetzt.

Soweit die Herstellung der Ausgangsverbindungen nicht beschrieben wird, sind diese bekannt oder analog zu bekannten Verbindungen oder hier beschriebenen Verfahren herstellbar. Es ist ebenfalls möglich, alle hier beschriebenen Umsetzungen in Parallel-Reaktoren oder mittels kombinatorischer Arbeitstechniken durchzuführen. Die Isomerengemische können nach üblichen Methoden wie beispielsweise Kristallisation, Chromatographie oder Salzbildung in die Enantiomeren bzw. E/Z-Isomeren aufgetrennt werden.
Die Herstellung der Salze erfolgt in üblicher Weise, indem man eine Lösung der Verbindung der Formel I mit der äquivalenten Menge oder einem Überschuß einer Base oder Säure, die gegebenenfalls in Lösung ist, versetzt und den Niederschlag abtrennt oder in üblicher Weise die Lösung aufarbeitet.

### Beispiel 1

### 5-Amino-1-(2-pyridinyl)-3-(3-pyridinylamino)-1H-1,2,4-triazol

527 mg N¹-Cyano-N²-(3-pyridyl)-O-phenyl-isoharnstoff werden in 10 ml Methanol gelöst, mit 295 mg 2-Hydrazinopyridin versetzt und 10 Stunden unter Rückfluss erhitzt. Nach dem Abkühlen wird der Niederschlag abgesaugt, mit Methanol gewaschen und getrocknet. Man erhält 362 mg (63,5 % der Theorie) 5-Amino-1-(2-pyridinyl)-3-(3-pyridinylamino)-1H-1,2,4-triazol vom Schmelzpunkt 256 °C.

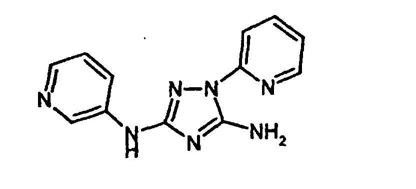

### Beispiel 2

### 5-Amino-1-(2-pyridinyl)-3-(2-pyridinylamino)-1H-1,2,4-triazol

97 mg N¹-Cyano-N²-(2-pyridyl)-O-phenyl-isoharnstoff werden in 2 ml Methanol gelöst, mit 51 mg 2-Hydrazinopyridin versetzt und 24 Stunden unter Rückfluss erhitzt. Nach dem Abkühlen wird der Niederschlag abgesaugt, mit Methanol gewaschen und getrocknet. Man erhält 15 mg (14,7 % der Theorie) 5-Amino-1-(2-pyridinyl)-3-(2-pyridinylamino)-1H-1,2,4-triazol vom Schmelzpunkt 332 °C.

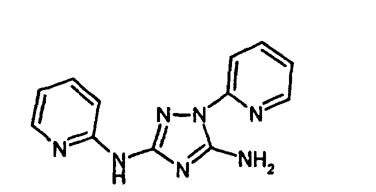

### Herstellung der Ausgangsverbindungen

1) N¹-Cyano-N²-(2-pyridyl)-O-phenyl-isoharnstoff
   443 mg 2-Aminopyridin und 953 mg Diphenylcyanocarbonimidat werden in 9,5 ml 1,4-Dioxan 5 Stunden bei 60 °C gerührt. Das Lösungsmittel wird abdestilliert und der Rückstand durch Säulenchromatographie über Kieselgel mit Methylenchlorid/-Ethanol (100:0 bis 95:5) gereinigt. Man erhält 555 mg Produkt vom Schmelzpunkt 105 °C.
2) N¹-Cyano-N²-(3-pyridyl)-O-phenyl-isoharnstoff
   443 mg 3-Aminopyridin und 953 mg Diphenylcyanocarbonimidat werden in 9,5 ml Isopropanol 24 Stunden bei Raumtemperatur gerührt. Der Niederschlag wird abgesaugt, mit Isopropanol gewaschen und getrocknet. Man erhält 527 mg N¹-Cyano-N²-(2-pyridyl)-O-phenyl-isoharnstoff vom Schmelzpunkt 146 °C.

In analoger Verfahrensweise werden auch folgende Verbindungen hergestellt:

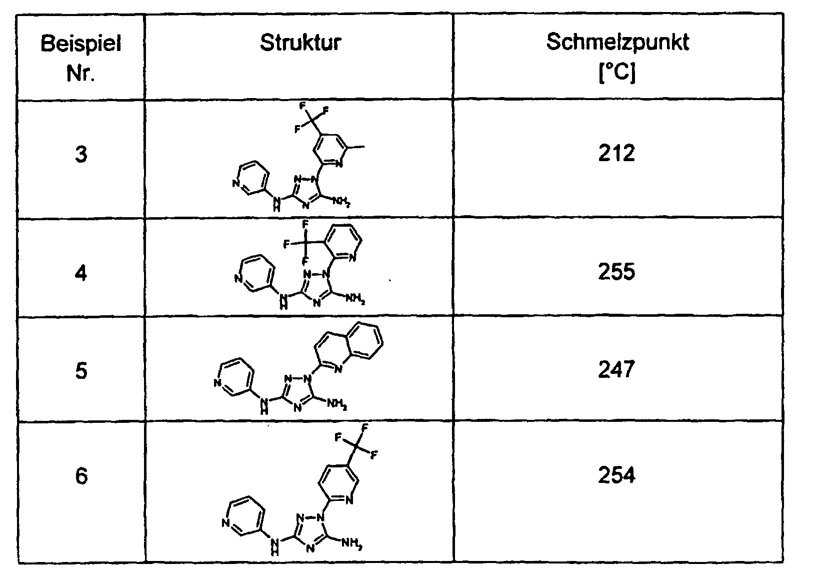

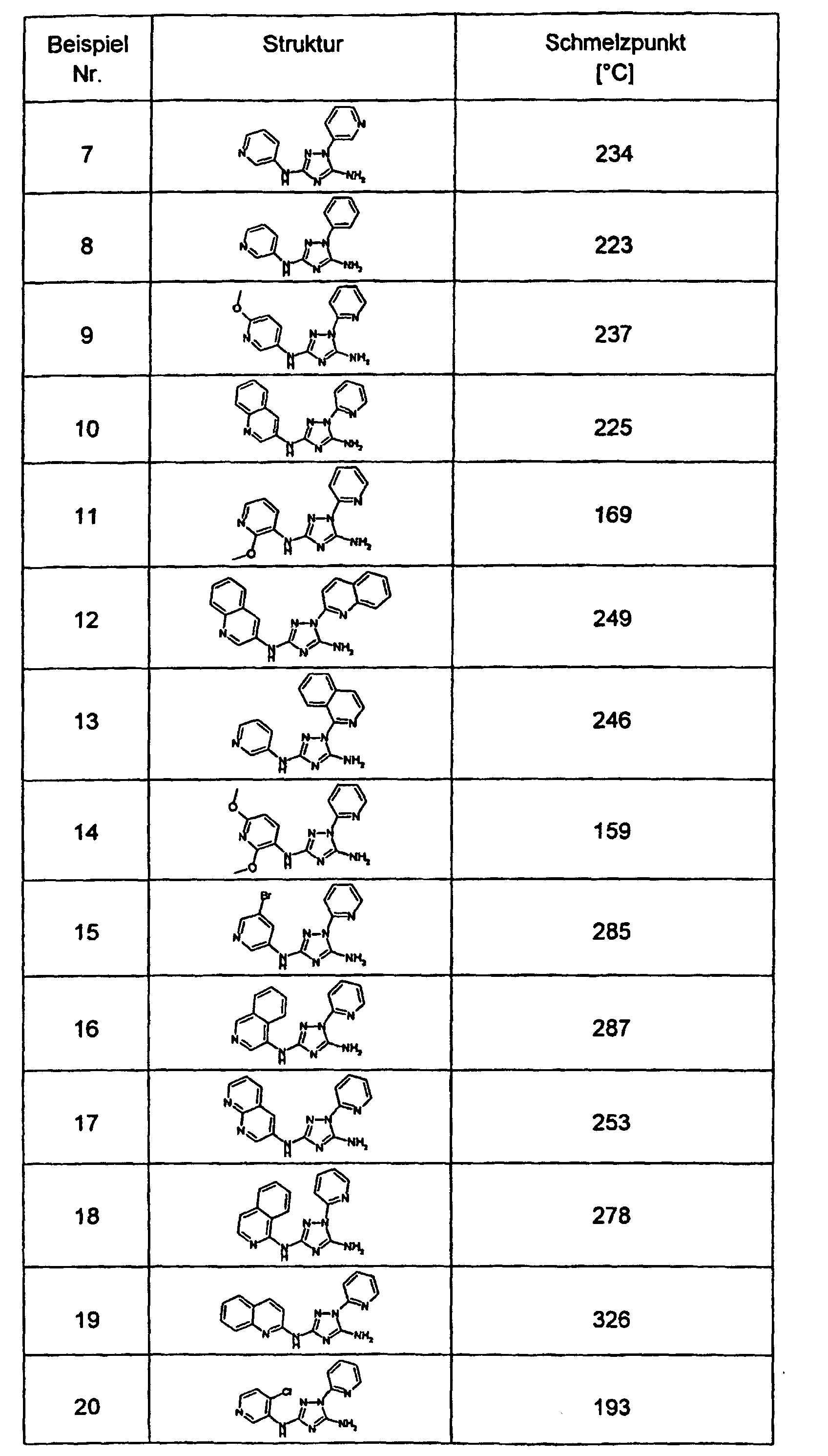

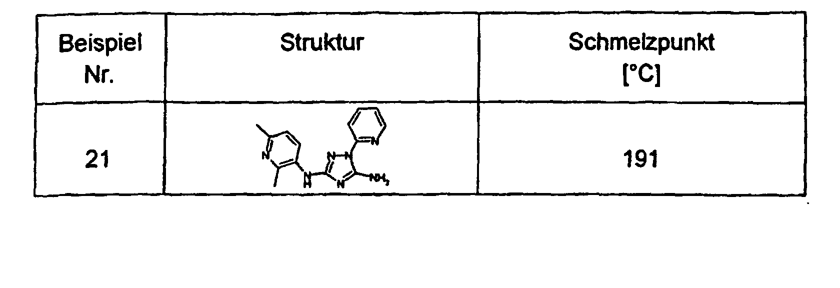

Die erfindungsgemäßen Verbindungen inhibieren im wesentlichen cyclin-abhängige Kinasen, worauf auch deren Wirkung zum Beispiel gegen Krebs, wie solide Tumoren und Leukämie, Autoimmunerkrankungen wie Psoriasis, Alopezie, und Multiple Sklerose, Chemotherapeutika-induzierte Alopezie und Mukositis, kardiovaskulare Erkrankungen, wie Stenosen, Arteriosklerosen und Restenosen, infektiöse Erkrankungen, wie z. B. durch unizellulare Parasiten, wie Trypanosoma, Toxoplasma oder Plasmodium, oder durch Pilze hervorgerufen, nephrologische Erkrankungen, wie z. B. Glomerulonephritis, chronische neurodegenerative Erkrankungen, wie Huntington's Erkrankung, amyotropisch laterale Sklerose, Parkinsonsche Erkrankung, AIDS Dementia und Alzheimer'sche Erkrankung, akute neurodegenerative Erkrankungen, wie Ischämien des Gehirns und Neurotraumata, virale Infektionen, wie z. B. Cytomegalus-Infektionen, Herpes, Hepatitis B und C, und HIV Erkrankungen basiert.

Der eukaryote Zellteilungszyklus stellt die Duplikation des Genoms und seine Verteilung auf die Tochterzellen sicher, indem er eine koordinierte und regulierte Abfolge von Ereignissen durchläuft. Der Zellzyklus wird in vier aufeinanderfolgende Phasen eingeteilt: Die G1 Phase repräsentiert die Zeit vor der DNA-Replikation, in der die Zelle wächst und für externe Stimuli empfänglich ist. In der S Phase repliziert die Zelle ihre DNA, und in der G2 Phase bereitet sie sich auf den Eintritt in die Mitose vor. In der Mitose (M Phase) wird die replizierte DNA getrennt und die Zellteilung vollzogen.

Die Zyklin-abhängigen Kinasen (CDKs), eine Familie von Ser/Thr-Kinasen, deren Mitglieder die Bindung eines Zyklins (Cyc) als regulatorische Untereinheit zu ihrer Aktivierung benötigen, treiben die Zelle durch den Zellzyklus. Unterschiedliche CDK/Cyc Paare sind in den verschiedenen Phasen des Zellzyklus aktiv. Für die grundlegende Funktion des Zellzyklus bedeutende CDK/Cyc Paare sind beispielsweise CDK4(6)/CycD, CDK2/CycE, CDK2/CycA, CDK1/CycA und CDK1/CycB. Einige Mitglieder der CDK-Enzymfamilie haben eine regulatorische Funktion, indem sie die Aktivität der vorgenannten Zellzyklus-CDKs beeinflussen, während anderen Mitgliedern der CDK-Enzymfamlie noch keine bestimmte Funktion zugeordnet werden konnte. Eine von diesen, CDK5, zeichnet sich dadurch aus, daß sie eine atypische, von den Zyklinen abweichende, regulatorische Untereinheit besitzt (p35), und ihre Aktivität im Gehirn am höchsten ist.

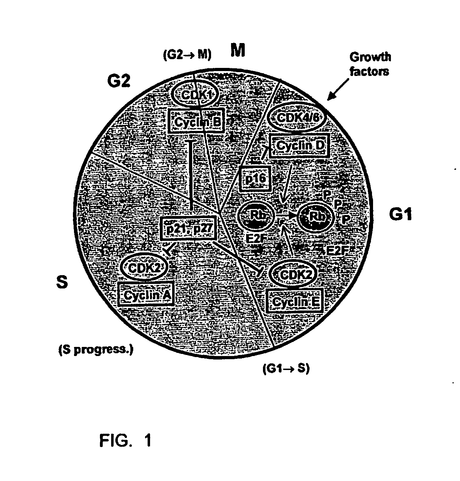

Der Eintritt in den Zellzyklus und das Durchlaufen des "Restriction Points", der die Unabhängigkeit einer Zelle von weiteren Wachstumssignalen für den Abschluß der begonnenen Zellteilung markiert, werden durch die Aktivität der CDK4(6)/CycD und CDK2/CycE Komplexe kontrolliert. Das wesentliche Substrat dieser CDK-Komplexe ist das Retinoblastoma-Protein (Rb), das Produkt des Retinoblastoma Tumorsuppressor Gens. Rb ist ein transkriptionelles Ko-Repressor Protein. Neben anderen noch weitgehend unverstandenen Mechanismen, bindet und inaktiviert Rb Transkriptionsfaktoren vom E2F-Typ, und bildet transkriptionelle Repressorkomplexe mit Histon-Deacetylasen (HDAC) (Zhang H.S. et al. (2000). Exit from G1 and S. phase of the cell cycle is regulated by repressor complexes containing HDAC-RbhSWI/SNF and Rb-hSWI/SNF. Cell 101, 79-89). Durch die Phosphorylierung des Rb durch CDKs werden gebundene E2F Transkriptionsfaktoren freigesetzt und führen zu transkriptioneller Aktivierung von Genen, deren Produkte für die DNA Synthese und die Progression durch die S-Phase benötigt werden. Zusätzlich bewirkt die Rb-Phosphorylierung die Auflösung der Rb-HDAC Komplexe, wodurch weitere Gene aktiviert werden. Die Phosphorylierung von Rb durch CDK's ist mit dem Überschreiten des "Restriction Points" gleichzusetzen. Für die Progression durch die S-Phase und deren Abschluß ist die Aktivität der CDK2/CycE und CDK2/CycA Komplexe notwendig, z. B. wird die Aktivität der Transkriptionsfaktoren vom E2F-Typ mittels Phosphorylierung durch CDK2/CycA abgeschaltet, sobald die Zellen in die S-Phase eingetreten sind. Nach vollständiger Replikation der DNA steuert die CDK1 im Komplex mit CycA oder CycB den Eintritt und das Durchlaufen der Phasen G2 und M (Abb. 1).

Entsprechend der außerordentlichen Bedeutung des Zellteilungszyklus ist das Durchlaufen des Zyklus streng reguliert und kontrolliert. Die Enzyme, die für die Progression durch den Zyklus notwendig sind, müssen zu dem richtigen Zeitpunkt aktiviert werden, und auch wieder abgeschaltet werden sobald die entsprechende Phase durchlaufen ist. Entsprechende Kontrollpunkte ("Checkpoints") arretieren die Progression durch den Zellzyklus, falls DNA-Schäden detektiert werden, oder die DNA-Replikation, oder der Aufbau des Spindelapparates noch nicht beendet ist.

Die Aktivität der CDKs wird durch verschiedene Mechanismen, wie Synthese und Degradation der Zykline, Komplexierung der CDKs mit den entsprechenden Zyklinen, Phosphorylierung und Dephosphorylierung regulatorischer Thr- und Tyr-Reste, und die Bindung natürlicher inhibitorischer Proteine, direkt kontrolliert. Während die Proteinmenge der CDKs in einer proliferierenden Zelle relativ konstant ist, oszilliert die Menge der einzelnen Zykline mit dem Durchlaufen des Zyklus. So wird zum Beispiel die Expression von CycD während der frühen G1 Phase durch Wachstumsfaktoren stimuliert, und die Expression von CycE wird nach Überschreiten des "Restriktion Points" durch die Aktivierung der Transkriptionsfaktoren vom E2F-Typ induziert. Die Zykline selbst werden durch Ubiquitin-vermittelte Proteolyse abgebaut. Aktivierende und inaktivierende Phosphorylierungen regulieren die Aktivität der CDK's, zum Beispiel phosphorylieren CDK-aktivierende Kinasen (CAKs) Thr160/161 der CDK1, wohingegen die Familie der Wee1/Myt1 Kinasen CDK1 durch Phosphorylierung von Thr14 und Tyr15 inaktivieren. Diese inaktivierenden Phosphorylierungen können durch cdc25 Phosphatasen wieder aufgehoben werden. Sehr bedeutsam ist die Regulation der Aktivität der CDK/Cyc-Komplexe durch zwei Familien natürlicher CDK Inhibitorproteine (CKIs), den Proteinprodukten der p21 Genfamilie (p21, p27, p57) und der p16 Genfamilie (p15, p16, p18, p19). Mitglieder der p21 Familie binden an Zyklin-Komplexe der CDKs 1,2,4,6, inhibieren aber nur Komplexe die CDK1 oder CDK2 enthalten. Mitglieder der p16 Familie sind spezifische Inhibitoren der CDK4- und CDK6-Komplexe.

Oberhalb dieser komplexen direkten Regulation der Aktivität der CDKs liegt die Ebene der Kontrollpunkt-Regulation. Kontrollpunkte erlauben der Zelle das geordnete Ablaufen der einzelnen Phasen während des Zellzykluses zu verfolgen. Die wichtigsten Kontrollpunkte liegen am Übergang von G1 nach S und von G2 nach M. Der G1-Kontrollpunkt stellt sicher, daß die Zelle keine DNA-Synthese beginnt falls sie nicht entsprechend ernährt ist, mit anderen Zellen oder dem Substrat korrekt interagiert, und ihre DNA intakt ist. Der G2/M Kontrollpunkt stellt die vollständige Replikation der DNA und den Aufbau der mitotischen Spindel sicher, bevor die Zelle in die Mitose eintritt. Der G1 Kontrollpunkt wird von dem Genprodukt des p53 Tumorsuppressorgens aktiviert. p53 wird nach Detektion von Veränderungen im Metabolismus oder der genomischen Integrität der Zelle aktiviert und kann entweder einen Stopp der Zellzyklusprogression oder Apoptose auslösen. Dabei spielt die transkriptionelle Aktivierung der Expression des CDK Inhibitorproteins p21 durch p53 eine entscheidende Rolle. Ein zweiter Zweig des G1 Kontrollpunktes umfaßt die Aktivierung der ATM und Chk1 Kinasen nach DNA-Schädigung durch UV-Licht oder ionisierende Strahlung und schließlich die Phosphorylierung und den nachfolgenden proteolytischen Abbau der cdc25A Phosphatase (Mailand N. et al. (2000). Rapid destruction of human cdc25A in response to DNA damage. Science 288, 1425-1429). Daraus resultiert eine Arretierung des Zellzykluses, da die inhibitorische Phosphorylierung der CDKs nicht entfernt wird. Nach Aktivierung des G2/M Kontrollpunktes durch Schädigung der DNA sind beide Mechanismen in ähnlicher Weise daran beteiligt, die Progression durch den Zellzyklus zu stoppen.

Der Verlust der Regulation des Zellzyklusses und der Verlust der Funktion der Kontrollpunkte sind Charakteristika von Tumorzellen. Der CDK-Rb-Signalweg ist in über 90% humaner Tumorzellen von Mutationen betroffen. Diese Mutationen, die schließlich zur inaktivierenden Phosphorylierung des RB führen, schließen die Überexpression von D- und E-Zyklinen durch Genamplifikation oder chromosomale Translokationen, inaktivierende Mutationen oder Deletionen von CDK-Inhibitoren des p16-Typs, sowie erhöhten (p27) oder verminderten (CycD) Proteinabbau ein. Die zweite Gruppe von Genen, die durch Mutationen in Tumorzellen getroffen sind,. kodiert für Komponenten der Kontrollpunkte. So ist p53, das essentiell für die G1 und G2/M Kontrollpunkte ist, das am häufigsten mutierte Gen in humanen Tumoren (ca. 50%). In Tumorzellen, die p53 ohne Mutation exprimieren, wird es häufig aufgrund einer stark erhöhten Proteindegradation inaktiviert. In ähnlicher Weise sind die Gene anderer für die Funktion der Kontrollpunkte notwendiger Proteine von Mutationen betroffen, zum Beispiel ATM (inaktivierende Mutationen) oder cdc25 Phosphatasen (Überexpression).

Überzeugende experimentelle Daten deuten darauf hin, daß CDK2/Cyc-Komplexe eine entscheidende Position während der Zellzyklusprogression einnehmen: (1) Sowohl dominant-negative Formen der CDK2, wie die transkriptionelle Repression der CDK2 Expression durch anti-sense Oligonukleotide bewirken einen Stopp der Zellzyklusprogression. (2) Die Inaktivierung des CycA Gens in Mäusen ist letal. (3) Die Störung der Funktion des CDK2/CycA Komplexes in Zellen mittels zellpermeabler Peptide führte zur Tumorzell-selektiven Apoptose (Chen Y.N.P. et al. (1999). Selective killing of transformed cells by cyclin/cyclin-dependent kinase 2 antagonists. Proc. Natl. Acad. Sci. USA 96, 4325-4329).

Veränderungen der Zellzykluskontrolle spielen nicht nur bei Krebserkrankungen eine Rolle. Der Zellzyklus wird durch eine Reihe von Viren, sowohl durch transformierende wie durch nicht-transformierende, aktiviert, um die Vermehrung der Viren in der Wirtszelle zu ermöglichen. Der fälschliche Eintritt in den Zellzyklus von normalerweise post-mitotischen Zellen wird mit verschiedenen neurodegenerativen Erkrankungen in Zusammenhang gebracht.
Die Mechanismen der Zellzyklusregulation, ihrer Veränderungen in Krankheiten und eine Vielzahl von Ansätzen zur Entwicklung von Inhibitoren der Zellzyklusprogression und speziell der CDKs wurden bereits in mehreren Publikationen ausführlich zusammenfassend beschrieben (Sielecki T.M. et al. (2000). Cyclin-dependent kinase inhibitors: useful targets in cell cycle regulation. J. Med. Chem. 43, 1-18; Fry D.W. & Garrett M.D. (2000). Inhibitors of cyclin-dependent kinases as therapeutic agents for the treatment of cancer. Curr. Opin. Oncol. Endo. Metab. Invest. Drugs 2, 40-59; Rosiania G.R. & Chang Y.T. (2000). Targeting hyperproliferative disorders with cyclin dependent kinase inhibitors. Exp. Opin. Ther. Patents 10, 215-230; Meijer L. et al. (1999). Properties and potential applications of chemical inhibitors of cyclin-dependent kinases. Pharmacol. Ther. 82, 279-284; Senderowicz A.M. & Sausville E.A. (2000). Preclinical and clinical development of cyclin-dependent kinase modulators. J. Natl. Cancer Inst. 92, 376-387).

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel Wasser, Gelantine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer.

Diese pharmazeutischen Präparate sind ebenfalls Gegenstand der vorliegenden Erfindung.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposomen oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die enteralen, parenteralen und oralen Applikationen sind ebenfalls Gegenstand der vorliegenden Erfindung.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1000 mg, vorzugsweise 50-200 mg, wobei die Dosis als einmal zu verabreichende Einzeldosis oder unterteilt in 2 oder mehreren Tagesdosen gegeben werden kann.
Ebenfalls Gegenstand der vorliegenden Erfindung ist die Verwendung der Verbindungen der allgemeinen Formel I, zur Herstellung eines Arzneimittels zur Behandlung von Krebs, Autoimmunerkrankungen, kardiovaskularen Erkrankungen, Chemotherapeutika-induzierter Alopezie und Mukositis, infektiösen Erkrankungen, nephrologischen Erkrankungen, chronischen und akuten neurodegenerativen Erkrankungen und viralen Infektionen, wobei unter Krebs solide Tumoren und Leukämie, unter Autoimmunerkrankungen Psoriasis, Alopezie und Multiple Sklerose, unter kardiovaskularen Erkrankungen Stenosen, Arteriosklerosen und Restenosen, unter infektiösen Erkrankungen durch unizellulare Parasiten hervorgerufene Erkrankungen, unter nephrologischen Erkrankungen Glomerulonephritis, unter chronisch neurodegenerativen Erkrankungen Huntington's Erkrankung, amyotropisch laterale Sklerose, Parkinsonsche Erkrankung, AIDS Dementia und Alzheimer'sche Erkrankung, unter akut neurodegenerativen Erkrankungen Ischämien des Gehirns und Neurotraumata, und unter viralen Infektionen Cytomegalus-Infektionen, Herpes, Hepatitis B oder C, und HIV Erkrankungen zu verstehen sind.

Ebenfalls Gegenstand der vorliegenden Erfindung sind Arzneimittel zur Behandlung der oben aufgeführten Erkrankungen, die mindestens eine Verbindung gemäß der allgemeinen Formel I enthalten, sowie Arzneimittel mit geeigneten Formulierungs- und Trägerstoffen.

Die erfindungsgemäßen Verbindungen der allgemeinen Formel I sind unter anderem hervorragende Inhibitoren der cyclin-abhängigen Kinasen, wie CDK1, CDK2, CDK3, CDK4, CDK5, CDK6, CDK7, CDK8 und CDK9, sowie der Glycogen-Synthase-Kinase (GSK-3β).

Die nachfolgenden Anwendungsbeispiele beschreiben die biologische Wirkung der erfindungsgemäßen Verbindungen ohne die Erfindung auf diese Beispiele zu beschränken.

### Anwendungsbeispiel 1

### CDK2/CycE Kinase Assay

Rekombinante CDK2- und CycE-GST-Fusionsproteine, gereinigt aus Bakulovirusinfizierten Insektenzellen (Sf9), wurden von Dr. Dieter Marmé, Klinik für Tumorbiologie Freiburg, erhalten. Histon IIIS, das als Kinase-Substrat verwendet wurde, wurde bei der Fa. Sigma gekauft.
CDK2/CycE (50 ng/Meßpunkt) wurde für 15 min bei 22°C in Anwesenheit verschiedener Konzentrationen an Testsubstanzen (0 µM, sowie innerhalb des Bereiches 0,01 - 100 µM) in Assaypuffer [50 mM Tris/HCl pH8,0, 10 mM MgCl₂, 0,1 mM Na ortho-Vanadat, 1,0 mM Dithiothreitol, 0,5 µM Adenosintrisphosphat (ATP), 10 µg/Meßpunkt Histon IIIS, 0,2 µCi/Meßpunkt ³³P-gamma ATP, 0,05% NP40, 12,5% Dimethylsulfoxid] inkubiert. Die Reaktion wurde durch Zugabe von EDTA-Lösung (250 mM, pH8,0, 14 µl/Meßpunkt) gestoppt.
Von jedem Reaktionsansatz wurden 10 µl auf P30 Filterstreifen (Fa. Wallac) aufgetragen, und nicht-eingebautes ³³P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex™ A, Fa. Wallac) bedeckt und für 1 Stunde bei 90°C eingebrannt. Die Menge an eingebautem ³³P (Substratphosphorylierung) wurde durch Szintillationsmessung in einem gamma-Strahlungsmeßgerät (Wallac) bestimmt.

### Anwendungsbeispiel 2

### KDR (VEGFR II) und Tie2 Assay

In einer spitz zulaufenden Mikrotiterplatte (ohne Proteinbindung!) werden die Lösungen in folgender Reihenfolge gemischt:
10 µl Substratmischung
10 µl Hemmstoffverdünnung
10 µl Enzymlösung

Man mischt gründlich und inkubiert 10 min bei Raumtemperatur. Danach gibt man 10 µl der Stop-Lösung zu, mischt und übertragt 10 µl der Lösung auf einen P 81, Phosphozellulosefilter. Man wäscht mehrfach in 0,1 M Phosphorsäure. Das Filterpapier wird getrocknet, mit Meltilex beschichtet und im Microbeta gemessen. Die IC50-Werte bestimmen sich aus der Inhibitorkonzentration, die notwendig ist, um den Phosphateinbau auf 50% des ungehemmten Einbaus nach Abzug des Leerwertes (EDTA-gestoppte Reaktion) zu hemmen.

### Für den Test verwendete Lösungen

**Stammlösungen** (Aliquote bei -70°C eingefroren)
A: 3 mM ATP in Wasser pH 7.0 (-70°C)
B: γ-³³P-ATP 1 mCi/100 µl
C: poly-(Glu₄Tyr) 10 mg/ml in Wasser

### Lösemittel für Verdünnungen:

Substratlösemittel: 10 mM DTT, 10 mM MnCl₂, 100 mM MgCl₂ (DTT oxidiert
leicht, bei -80°C einfrieren). Vor dem Versuch 1:10 mit H₂O verdünnen. Enzymlösemittel: 120mM TrisCl pH 7.5; 10 µM Na₃VO₄

### Arbeitslösungen:

Pro 125 Ansätze werden folgende Mengen benötigt:

### Substratmix:

10µl Vol ATP Stammlösung A +25 µCi γ-³³P-ATP (ca 2,5 µl der Stammlösung B) + 30µl poly-(Glu₄Tyr) Stammlösung C+ 1,21 ml Substratlösemittel
Hemmstofflösung:
   Substanzen entsprechend den Verdünnungen, als Kontrolle 3% DMSO in
   Substratlösemittel
Enzymlösung:
   11,25 µg der Enzymstammlösung (KDR und FLT-1 Kinase und Tie2) werden bei 4°C in 1,25 ml Enzymlösemittel verdünnt (Andere Mengen bei anderer Aktivität!).
Stopplösung: 250 mM EDTA pH 7.0
Waschlösung: 0,5% Phosphorsäure

Die Ergebnisse der Anwendungsbeispiele 1 und 2 sind in der folgenden Tabelle aufgeführt:

| Beispiel Nr. | VEGFR II IC₅₀ [µM] | CDK2 IC₅₀ [µM] | Tie 2 IC₅₀ [µM] |
|---|---|---|---|
| 3 | 0,01 | 2,0 | > 1,0 |
| 4 | 0,03 | 6,0 | > 1,0 |
| 5 | 0,01 | 0,4 | > 1,0 |
| 6 | 0,2 | 2,0 | > 1,0 |
| 9 | 0,05 | 1,0 | 0,5 |
| 10 | 0,02 | 0,8 | 0,5 |
| 13 | 0,02 | 0,2 | 0,5 |
| 15 | 0,04 | 0,3 | 0,5 |
| 16 | 0,02 | 2,8 | 1,0 |
| 17 | 0,03 | 0,5 | >1,0 |

### Anwendungsbeispiel 3

### Proliferationsassay

Kultivierte humane MCF7 Tumorzellen wurden in einer Dichte von 5000 Zellen/Meßpunkt in einer 96-well Multititerplatte in 200 µl des entsprechenden Wachstumsmediums ausplattiert. Nach 24 Stunden wurden die Zellen einer Platte (Nullpunkt-Platte) mit Kristallviolett gefärbt (s.u.), während das Medium der anderen Platten durch frisches Kulturmedium (200 µl), dem die Testsubstanzen in verschiedenen Konzentrationen (0 µM, sowie im Bereich 0,01 - 30 µM; die finale Konzentration des Lösungsmittels Dimethylsulfoxid betrug 0,5%) zugesetzt waren, ersetzt. Die Zellen wurden für 4 Tage in Anwesenheit der Testsubstanzen inkubiert. Die Zellproliferation wurde durch Färbung der Zellen mit Kristallviolett bestimmt: Die Zellen wurden durch Zugabe von 20 µl/Meßpunkt einer 11 %igen Glutaraldehyd-Lösung 15 min bei Raumtemperatur fixiert. Nach dreimaligem Waschen der fixierten Zellen mit Wasser wurden die Platten bei Raumtemperatur getrocknet. Die Zellen wurden durch Zugabe von 100 µl/Meßpunkt einer 0,1%igen Kristallviolett-Lösung (pH durch Zugabe von Essigsäure auf pH3 eingestellt) gefärbt. Nach dreimaligem Waschen der gefärbten Zellen mit Wasser wurden die Platten bei Raumtemperatur getrocknet. Der Farbstoff wurde durch Zugabe von 100 µl/Meßpunkt einer 10%igen Essigsäure-Lösung gelöst. Die Extinktion wurde photometrisch bei einer Wellenlänge von 595 nm bestimmt. Die prozentuale Änderung des Zellwachstums wurde durch Normalisierung der Meßwerte auf die Extinktionwerte der Nullpunktplatte (=0%) und die Extinktion der unbehandelten (0 µM) Zellen (=100%) berechnet.

Die Ergebnisse sind in der nachfolgenden Tabelle aufgeführt:

| **Beispiel Nr.** | **MCF-7 IC 50 [µM]** |
|---|---|
| 1 | 1,5 |
| 3 | >10 |
| 4 | 6 |
| 5 | 2 |
| 6 | 4 |
| 9 | >10 |
| 10 | 4 |
| 13 | 0,7 |
| 15 | 1,1 |
| 17 | 1,5 |

## Patentansprüche

1. Verbindungen der allgemeinen Formel I 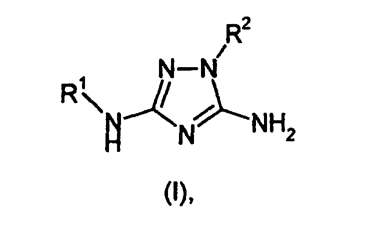 in der
R¹ für mono- oder bicyclisches Heteroaryl, welches gegebenfalls ein- oder mehrfach, gleich oder verschieden substituiert sein kann und
R² für mono- oder bicyclisches Aryl oder Heteroaryl, welches gegebenfalls ein- oder mehrfach, gleich oder verschieden substituiert sein kann, stehen, sowie deren Isomeren und Salze,

2. Verbindungen der allgemeinen Formel I, gemäß Anspruch 1, in der
R¹ für die Gruppe 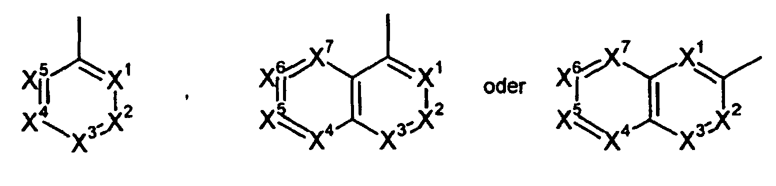 steht, worin
X¹ bis X⁷ unabhängig voneinander für ein Stickstoff-Atom oder für die Gruppe CR³ stehen, wobei mindestens ein X im Ring für ein Stickstoff-Atom steht und
R³ für Wasserstoff, Hydroxy, Halogen, C₁-C₁₀-Alkyl, C₁-C₁₀-Alkoxy, Halogen-C₁-C₁₀-alkyl, Halogen-C₁-C₁₀-alkoxy, Aryl, Aryloxy, Heteroaryl, Heteroaryloxy oder für die Gruppe -NR⁴R⁵ steht,
R² für die Gruppe 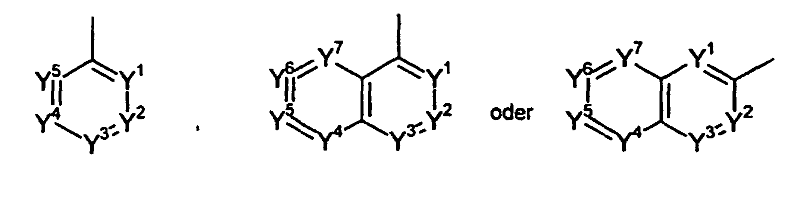 steht, worin
Y¹ bis Y⁷ unabhängig voneinander für ein Stickstoff-Atom oder für die Gruppe CR⁶ stehen und
R⁶ für Wasserstoff, Hydroxy, Halogen, Nitro, Cyano, C₁-C₁₀-Alkyl, C₁-C₁₀-Alkoxy, Halogen-C₁-C₁₀-alkyl, Halogen-C₁-C₁₀-alkoxy, Aryl, Aryloxy, Heteroaryl, Hetaroaryloxy, C₁-C₁₀-Alkylcarbonyl, C₁-C₁₀-Alkoxycarbonyl oder für die Gruppe -NR⁴R⁵ steht, bedeuten, sowie deren Isomeren und Salze.

3. Verbindungen der allgemeinen Formel I, gemäß den Ansprüchen 1 und 2, in der
R¹ für die Gruppe 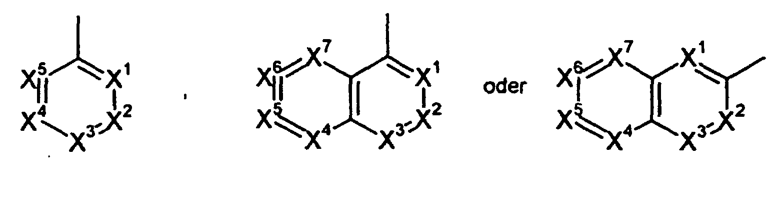 steht, worin
X¹ bis X⁷ unabhängig voneinander für ein Stickstoff-Atom oder für die Gruppe CR³ stehen, wobei mindestens ein X im Ring für ein Stickstoff-Atom steht und
R³ für Wasserstoff, Halogen, C₁-C₆-Alkyl oder C₁-C₆-Alkoxy steht,
R² für die Gruppe 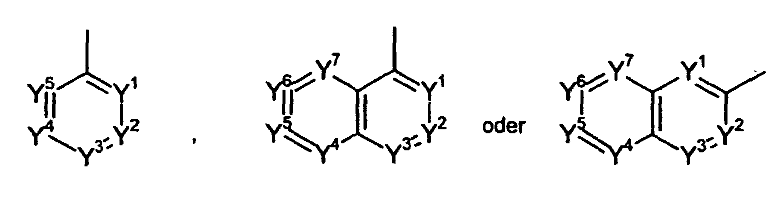 steht, worin
Y¹ bis Y⁷ unabhängig voneinander für ein Stickstoff-Atom oder für die Gruppe CR⁶ stehen und
R⁶ für Wasserstoff, C₁-C₆-Alkyl oder Halogen-C₁-C₆-alkyl steht, bedeuten, sowie deren Isomeren und Salze.

4. Verbindungen der allgemeinen Formel I, gemäß den Ansprüchen 1 bis 3, in der
R¹ für die Gruppe 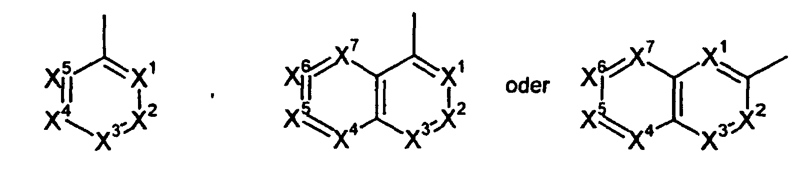 steht, worin
X¹ bis X⁴ unabhängig voneinander für ein Stickstoff-Atom oder für die Gruppe CR³ stehen, wobei mindestens ein X für ein Stickstoff-Atom steht,
X⁵ bis X⁷ für die Gruppe CR³ stehen,
R³ für Wasserstoff, Methoxy, Brom, Chlor oder Methyl steht,
R² für die Gruppe 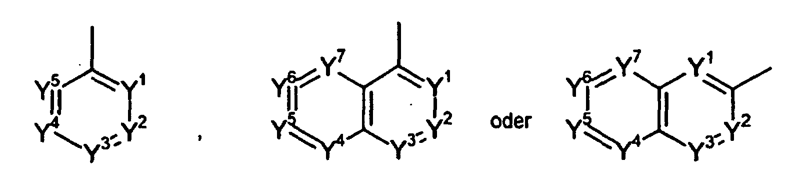 steht, worin
Y¹ bis Y⁷ unabhängig voneinander für ein Stickstoff-Atom oder für die Gruppe CR⁶ stehen und
R⁶ für Wasserstoff, Methyl oder Trifluormethyl steht, bedeuten, sowie deren Isomeren und Salze.

5. Verwendung der Verbindungen der allgemeinen Formel I, gemäß den Ansprüchen 1 bis 4, zur Herstellung eines Arzneimittels zur Behandlung von Krebs, Autoimmunerkrankungen, Chemotherapeutika-induzierter Alopezie und Mukositis, kardiovaskularen Erkrankungen, infektiösen Erkrankungen, nephrologischen Erkrankungen, chronisch und akut neurodegenerativen Erkrankungen und viralen Infektionen.

6. Verwendung gemäß Anspruch 5, **dadurch gekennzeichnet, daß** unter Krebs solide Tumoren und Leukämie, unter Autoimmunerkrankungen Psoriasis, Alopezie und Multiple Sklerose, unter kardiovaskularen Erkrankungen Stenosen, Arteriosklerosen und Restenosen, unter infektiösen Erkrankungen durch unizellulare Parasiten hervorgerufene Erkrankungen, unter nephrologischen Erkrankungen Glomerulonephritis, unter chronisch neurodegenerativen Erkrankungen Huntington's Erkrankung, amyotropisch laterale Sklerose, Parkinsonsche Erkrankung, AIDS Dementia und Alzheimer'sche Erkrankung, unter akut neurodegenerativen Erkrankungen Ischämien des Gehirns und Neurotraumata, und unter viralen Infektionen Cytomegalus-Infektionen, Herpes, Hepatitis B und C und HIV Erkrankungen zu verstehen sind.

7. Arzneimittel, enthaltend mindestens eine Verbindung gemäß den Ansprüchen 1 bis 4.

8. Arzneimittel gemäß Anspruch 7, zur Behandlung von Krebs, Autoimmunerkrankungen, kardiovaskularen Erkrankungen, infektiöse Erkrankungen, nephrologische Erkrankungen, neurodegenerative Erkrankungen und viralen Infektionen.

9. Arzneimittel gemäß Anspruch 8, **dadurch gekennzeichnet, daß** unter Krebs solide Tumoren und Leukämie, unter Autoimmunerkrankungen Psoriasis, Alopezie und Multiple Sklerose, unter kardiovaskularen Erkrankungen Stenosen, Arteriosklerosen und Restenosen, unter infektiösen Erkrankungen durch unizellulare Parasiten hervorgerufene Erkrankungen, unter nephrologischen Erkrankungen Glornerulonephritis, unter chronisch neurodegenerativen Erkrankungen Huntington's Erkrankung, amyotropisch laterale Sklerose, Parkinsonsche Erkrankung, AIDS Dementia und Alzheimer'sche Erkrankung, unter akut neurodegenerativen Erkrankungen Ischämien des Gehirns und Neurotraumata, und unter viralen Infektionen Cytomegalus-Infektionen, Herpes, Hepatitis B und C und HIV Erkrankungen zu verstehen sind.

10. Verbindungen gemäß den Ansprüchen 1 bis 4 und Arzneimittel gemäß den Ansprüchen 7 bis 9 mit geeigneten Formulierungs- und Trägerstoffen.

## Claims

1. Compounds of the general formula I 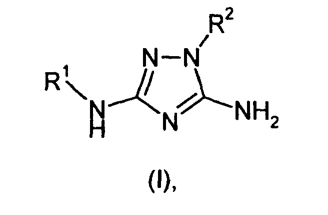 in which
R¹ is monocyclic or bicyclic heteroaryl which can be optionally substituted, once or more than once, identically or differently, and
R² is monocyclic or bicyclic aryl or heteroaryl which can be optionally substituted, once or more than once, identically or differently, and also their isomers and salts.

2. Compounds of the general formula I, according to Claim 1, in which
R¹ is the group 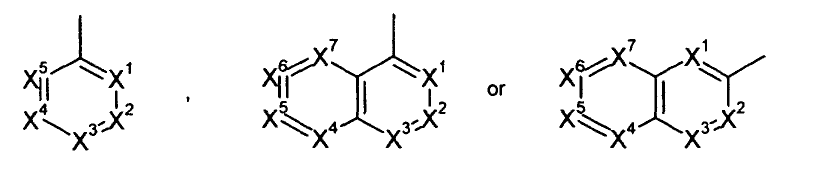 in which
X¹ to X⁷ are, independently of each other, a nitrogen atom or the group CR³, with at least one X in the ring being a nitrogen atom, and
R³ is hydrogen, hydroxyl, halogen, C₁-C₁₀- alkyl, C₁-C₁₀-alkoxy, halo-C₁-C₁₀-alkyl, halo-C₁-C₁₀-alkoxy, aryl, aryloxy, heteroaryl, heteroaryloxy or the group -NR⁴R⁵,
R² is the group 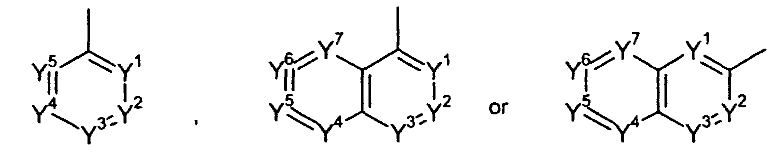 in which
Y¹ to Y⁷ are, independently of each other, a nitrogen atom or the group CR⁶, and
R⁶ is hydrogen, hydroxyl, halogen, nitro, cyano, C₁-C₁₀-alkyl, C₁-C₁₀-alkoxy, halo-C₁-C₁₀-alkyl, halo-C₁-C₁₀-alkoxy, aryl, aryloxy, heteroaryl, heteroaryloxy, C₁-C₁₀-alkylcarbonyl, C₁-C₁₀-alkoxycarbonyl or the group -NR⁴R⁵, and also their isomers and salts.

3. Compounds of the general formula I, according to Claims 1 and 2, in which
R¹ is the group 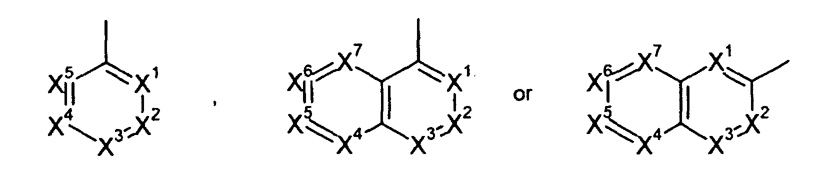 in which
X¹ to X⁷ are, independently of each other, a nitrogen atom or the group CR³, with at least one X in the ring being a nitrogen atom, and
R³ is hydrogen, halogen, C₁-C₆-alkyl or C₁-C₆-alkoxy,
R² is the group 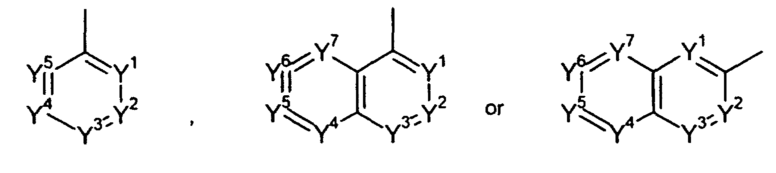 in which
Y¹ to Y⁷ are, independently of each other, a nitrogen atom or the group CR⁶, and
R⁶ is hydrogen, C₁-C₆-alkyl or halo-C₁-C₆-alkyl, and also their isomers and salts.

4. Compounds of the general formula I, according to Claims 1 to 3, in which
R¹ is the group 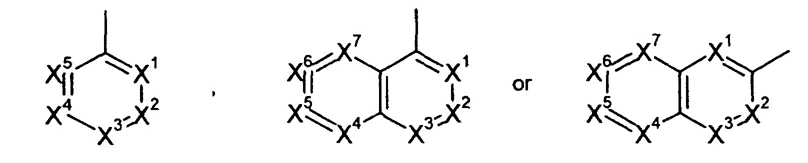 in which
X¹ to X⁴ are, independently of each other, a nitrogen atom or the group CR³, with at least one X being a nitrogen atom,
X⁵ to X⁷ are the group CR³,
R³ is hydrogen, methoxy, bromine, chlorine or methyl,
R² is the group 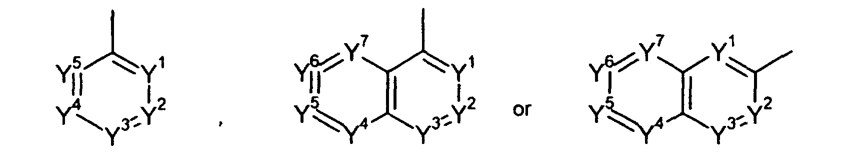 in which
Y¹ to Y⁷ are, independently of each other, a nitrogen atom or the group CR⁶, and
R⁶ is hydrogen, methyl or trifluoromethyl, and also their isomers and salts.

5. Use of the compounds of the general formula I, according to Claims 1 to 4, for producing a drug for treating cancer, autoimmune diseases, chemotherapeutic-induced alopecia and mucositis, cardiovascular diseases, infectious diseases, nephrologic diseases, chronic and acute neurodegenerative diseases and viral infections.

6. Use according to Claim 5, **characterized in that** cancer is to be understood as meaning solid tumours and leukaemia, autoimmune diseases to be understood as meaning psoriasis, alopecia and multiple sclerosis, cardiovascular diseases to be understood as meaning stenoses, arterioscleroses and restenoses, infectious diseases to be understood as meaning diseases caused by unicellular parasites, nephrologic diseases to be understood as meaning glomerulonephritis, chronic neurodegenerative diseases to be understood as meaning Huntington's disease, amyotrophic lateral sclerosis, Parkinson's disease, AIDS dementia and Alzheimer's disease, acute neurodegenerative diseases to be understood as meaning ischaemias of the brain and neurotraumas, and viral infections to be understood as meaning cytomegalus infections, herpes, hepatitis B and C and HIV diseases.

7. Drugs, comprising at least one compound according to Claims 1 to 4.

8. Drugs according to Claim 7, for treating cancer, autoimmune diseases, cardiovascular diseases, infectious diseases, nephrologic diseases, neurodegenerative diseases and viral infections.

9. Drugs according to Claim 8, **characterized in that** cancer is to be understood as meaning solid tumours and leukaemia, autoimmune diseases to be understood as meaning psoriasis, alopecia and multiple sclerosis, cardiovascular diseases to be understood as meaning stenoses, arterioscleroses and restenoses, infectious diseases to be understood as meaning diseases caused by unicellular parasites, nephrologic diseases to be understood as meaning glomerulonephritis, chronic neurodegenerative diseases to be understood as meaning Huntington's disease, amyotrophic lateral sclerosis, Parkinson's disease, AIDS dementia and Alzheimer's disease, acute neurodegenerative diseases to be understood as meaning ischaemias of the brain and neurotraumas, and viral infections to be understood as meaning cytomegalus infections, herpes, hepatitis B and C and HIV diseases.

10. Compounds according to Claims 1 to 4 and drugs according to Claims 7 to 9 comprising suitable formulation substances and carrier substances.

## Revendications

1. Composés de formule générale I 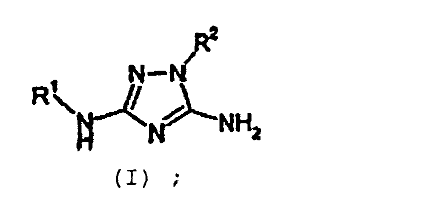 dans laquelle
R¹ représente un hétéroaryle mono- ou bicyclique, qui peut être éventuellement substitué une ou plusieurs fois, de manière identique ou différente, et
R² représente un aryle ou hétéroaryle mono- ou bicyclique, qui peut être éventuellement substitué une ou plusieurs fois, de manière identique ou différente, ainsi que leurs isomères et sels.

2. Composés de formule générale I, selon la revendication 1, dans laquelle
R¹ 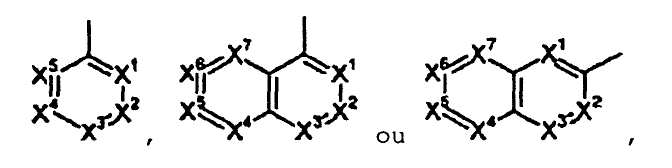
dans lequel
X¹ à X⁷ représentent, indépendamment les uns des autres, un atome d'azote, ou le groupe CR³, au moins un X dans le cycle représentant un atome d'azote et
R³ représentant un atome d'hydrogène, d' halogène, un hydroxy, alkyle en C₁ à C₁₀, alcoxy en C₁ à C₁₀, alkyle en C₁ à C₁₀ halogéné, alcoxy en C₁ à C₁₀ halogéné, aryle, aryloxy, hétéroaryle, hétéroaryloxy ou le groupe - NR⁴R⁵,
R² représente le groupe 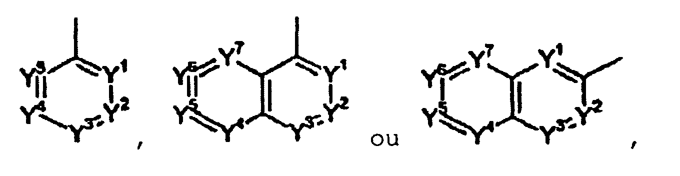
dans lequel
Y¹ à Y⁷ représentent, indépendamment les uns des autres, un atome d'azote ou le groupe CR⁶ et
R⁶ représente un atome d'hydrogène, un hydroxy, d'halogène, nitro, cyano, alkyle en C₁ à C₁₀, alcoxy en C₁ à C₁₀, alkyle en C₁ à C₁₀ halogéné, alcoxy en C₁ à C₁₀ halogéné, aryle, aryloxy, hétéroaryle, hétéroaryloxy, (alkyle en C₁ à C₁₀)carbonyle, (alcoxy en C₁ à C₁₀)carbonyle ou le groupe -NR⁴R⁵, ainsi que leurs isomères et sels.

3. Composés de formule générale I, selon les revendications 1 et 2. dans lesquels
R¹ représente le groupe 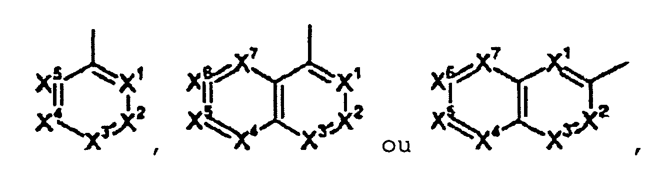
dans lequel
X¹ à X⁷ représentent, indépendamment les uns des autres, un atome d'azote, ou le groupe CR³, au moins un X dans le cycle représentant un atome d'azote et
R³ représentant un atome d'hydrogène, d'halogène, un alkyle en C₁ à C₆ ou alcoxy en C₁ à C₆,
R² représente le groupe 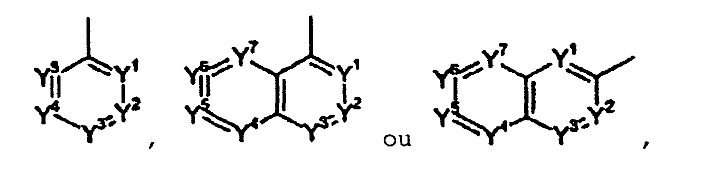
dans lequel
Y¹ à Y⁷ représentent, indépendamment les uns des autres, un atome d'azote ou le groupe CR⁶ et
R⁶ représente un atome d'hydrogène, un alkyle en C₁ à C₆ ou un alkyle en C₁ à C₆ halogéné, ainsi que leurs isomères et sels.

4. Composés de formule générale I, selon les revendications 1 à 3, dans lesquels
R¹ représente le groupe 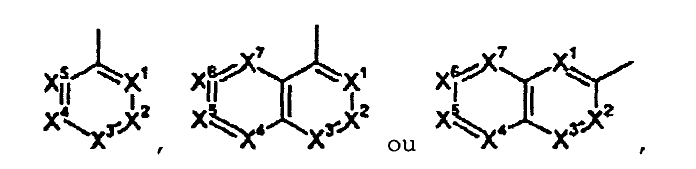
dans lequel
X¹ à X⁴ représentent, indépendamment les uns des autres, un atome d'azote, ou le groupe CR³, au moins un X dans le cycle représentant un atome d'azote,
X⁵ à X⁷ représentent le groupe CR³,
R³ représente un atome d'hydrogène un méthoxy, de brome, chlore, ou méthyle,
R² représente le groupe 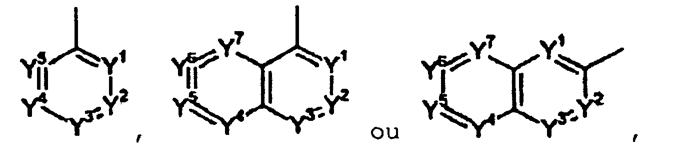
dans lequel
Y¹ à Y⁷ représentent, indépendamment les uns des autres, un atome d'azote ou le groupe CR⁶ et
R⁶ représente un atome d'hydrogène, un méthyle ou trifluorométhyle, ainsi que leurs isomères et sels.

5. Utilisation des composés de formule générale I, selon les revendications 1 à 4, pour la préparation d'un médicament pour le traitement du cancer, de maladies auto-immunes, d'alopécie et de mycose induites par une chimiothérapie, de maladies cardiovasculaires, de maladies infectieuses, de maladies néphrologiques, de maladies neurodégénératives chroniques et aiguës et d'infections virales.

6. Utilisation selon la revendication 5, **caractérisée en ce qu'**il faut entendre par cancer les tumeurs solides et la leucémie, par maladies auto-immunes le psoriasis, l'alopécie et la sclérose en plaques, par maladies cardiovasculaires les sténoses, les artérioscléroses et les resténoses, par maladies infectieuses les maladies dues à des parasites unicellulaires, par maladies néphrologiques la glomérulonéphrite, par maladies neurodégénératives chroniques la maladie de Huntington, la sclérose latérale amyotrophique, la maladie de Parkinson, la démence du SIDA et la maladie d'Alzheimer, par maladies neurodégénératives aiguës les ischémies cérébrales et les neurotraumatismes, et par infections virales les infections à cytomégalovirus. l'herpès, l'hépatite B et C et les maladies du VIH.

7. Médicament, contenant au moins un composé selon les revendications 1 à 4.

8. Médicament selon la revendication 7, pour le traitement de cancer, de maladies auto-immunes, de maladies cardiovasculaires, de maladies infectieuses, de maladies néphrologiques, de maladies neurodégénératives et d'infections virales.

9. Médicament selon la revendication 8, **caractérisé en ce qu'**il faut entendre par cancer les tumeurs solides et la leucémie, par maladies auto-immunes le psoriasis, l'alopécie et la sclérose en plaques, par maladies cardiovasculaires les sténoses, les artérioscléroses et les resténoses, par maladies infectieuses les maladies dues à des parasites unicellulaires, par maladies néphrologiques la glomérulonéphrite, par maladies neurodégénératives chroniques la maladie de Huntington, la sclérose latérale amyotrophique, la maladie de Parkinson, la démence du SIDA et la maladie d'Alzheimer, par maladies neurodégénératives aiguës les ischémies cérébrales et les neurotraumatismes, et par infections virales les infections à cytomégalovirus, l'herpès, l'hépatite B et C et les maladies du VIH.

10. Composés selon les revendications 1 à 4 et médicament selon les revendications 7 à 9 avec des substances de formulations et des véhicules appropriés.
